(19) Europäisches Patentamt
European Patent Office
Office européen des brevets

(11) **EP 4 458 266 A1**

(12) **EUROPEAN PATENT APPLICATION**
published in accordance with Art. 153(4) EPC

(43) Date of publication:
**06.11.2024  Bulletin 2024/45**

(21) Application number: **22916536.0**

(22) Date of filing: **09.12.2022**

(51) International Patent Classification (IPC):
*A61B 5/11* $^{(2006.01)}$     *A61B 5/00* $^{(2006.01)}$
*A61B 5/16* $^{(2006.01)}$     *G16H 50/20* $^{(2018.01)}$
*G16H 50/70* $^{(2018.01)}$

(52) Cooperative Patent Classification (CPC):
**A61B 5/4088; A61B 5/00; A61B 5/11;
A61B 5/1116; A61B 5/112; A61B 5/16;
A61B 5/4023; G16H 50/20; G16H 50/70;**
A61B 2562/0219

(86) International application number:
**PCT/KR2022/020054**

(87) International publication number:
**WO 2023/128391 (06.07.2023 Gazette 2023/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.12.2021  KR 20210194313**

(71) Applicants:
• **Plan B4U Co., Ltd.**
**Seongnam-si, Gyeonggi-do 13605 (KR)**

• **Seoul National University Hospital**
**Seoul 03080 (KR)**

(72) Inventors:
• **KIM, Ki Woong**
**Seoul 06718 (KR)**
• **LEE, Hyang Jun**
**Seoul 06356 (KR)**
• **BAE, Jong Bin**
**Seoul 05698 (KR)**

(74) Representative: **AWA Sweden AB**
**Box 45086**
**104 30 Stockholm (SE)**

(54) **METHOD AND DEVICE FOR ESTIMATING WALKING SPEED**

(57)  According to an embodiment, provided is a method of estimating a gait speed, the method including: determining N subjects for gait speed measurement and obtaining personal data of the subjects; performing a clinical assessment on the subjects; obtaining measured gait data by performing a gait assessment on the subjects; and developing a gait speed estimation model having a plurality of gait factors based on the measured gait data, wherein the gait speed estimation model includes, as gait factors, a roll angle, a yaw angle, and a body weight measured by using an accelerometer worn on a center of body mass of the subject.

FIG. 2

```
        ┌─────────┐
        │  START  │
        └────┬────┘
             │
  ┌──────────────────────────────┐
  │ COLLECT DATA REGARDING SUBJECTS │──── S10
  └──────────────┬───────────────┘
                 │
  ┌──────────────────────────────┐
  │  PERFORM CLINICAL ASSESSMENT   │──── S20
  └──────────────┬───────────────┘
                 │
  ┌──────────────────────────────┐
  │    PERFORM GAIT ASSESSMENT     │──── S30
  └──────────────┬───────────────┘
                 │
  ┌──────────────────────────────┐
  │ DEVELOP GAIT SPEED ESTIMATION MODEL │──── S40
  └──────────────┬───────────────┘
                 │
           ┌─────┴────┐
           │   END    │
           └──────────┘
```

EP 4 458 266 A1

## Description

## Technical Field

Embodiments relate to a method and device for estimating a gait speed.

## Background Art

Human gait changes with the degeneration of muscles and senses. Elderly people may have various age-related issues that alter their gait. In previous studies, human kinematics and muscle weakness are major concerns in altering a step length. People with many degenerative changes may have shorter leg lengths because their walking efficiency decreases with longer step lengths. In addition, there are studies indicating that both step length and body weight show a curvilinear relationship with gait speed.

Gait speed is associated with frailty, disability, falls, cognitive decline, and mortality. However, most previous studies manually measured gait speed by using a stopwatch, leading to human error and limited accuracy. Thus, laboratory-based motion capture systems and instrumented walkways have been developed, providing more accurate and reliable estimates of gait speed than manual measurements with a stopwatch. However, laboratory-based motion capture systems and instrumented walkways can only measure a limited number of steps due to spatial constraints and have the disadvantage of high initial costs.

To overcome these spatial and cost limitations, wearable acceleration sensors, which are cheaper and less spatially restricted than motion capture systems and instrumented walkways, and less sensitive to human error than stopwatches, may be used to estimate a gait speed. However, wearable acceleration sensors do not directly measure a gait speed but estimate it indirectly through acceleration, kinematic modeling, gait motion correction, data regression modeling, or hybrid methods, which leads to an issue that the accuracy of a gait speed estimated by an inertial sensor is not high.

## Disclosure

## Technical Problem

Provided are a method and device for accurately estimating a gait speed to diagnose aging and relevant risks.

Technical objectives of the present disclosure are not limited to the foregoing, and other unmentioned objectives or advantages of the present disclosure would be understood from the following description and be more clearly understood from the embodiments of the present disclosure. In addition, it would be appreciated that the objectives and advantages of the present disclosure may be implemented by means provided in the claims and a combination thereof.

## Technical Solution

According to an embodiment, provided is a method of estimating a gait speed, the method including: determining N subjects for gait speed measurement and obtaining personal data of the subjects; obtaining data regarding a result of performing a clinical assessment on the subjects; obtaining measured gait data by performing a gait assessment on the subjects; and developing a gait speed estimation model having a plurality of gait factors based on the measured gait data, wherein the gait speed estimation model includes, as gait factors, a roll angle, a yaw angle, and a body weight measured by using an accelerometer worn on a center of body mass of the subject.

In the present disclosure, the determining of the N subjects may include determining, as the N subjects, people in a population after excluding those who have diseases that is likely to affect a gait or whose scores of a performance-oriented mobility assessment are less than a preset score.

In the present disclosure, the clinical assessment may be to diagnose mental disorders according to pre-established diagnostic criteria, determine global severity of cognitive disorders, and evaluate gait and balance abilities by using Performance Oriented Mobility Assessment (POMA).

In the present disclosure, the obtaining of the measured gait data may include obtaining gait characteristic values of the subject by using the accelerometer attached to the center of body weight and an existing gait measurement device.

In the present disclosure, the accelerometer may be an inertial measurement unit (IMU) attached to waist of the subject with a predetermined adhesive material, and may be a device capable of measuring three-axis acceleration.

In the present disclosure, the developing of the gait speed estimation model may include: a first model that performs linear regression analysis including roll angle, yaw angle, and body weight as gait factors; and a second model that classifies the subjects into a plurality of subgroups based on normalized step length (NSL), and performs the linear

regression analysis for each subgroup.

In the present disclosure, the NSL may be calculated according to an equation

$$NSL = \frac{l_s}{h} \approx \frac{2l_1}{h}\beta_1(0)\sin(a) = 2\alpha_1\beta_1(0)\sin(a)$$

, and in the above equation, $l_1$ may denote a leg length, $l_s$ may denote a step length, $\alpha_1$ may denote a human leg/height ratio, $\beta_1$ may denote a coefficient of leg bending, and a may denote an angular displacement.

In the present disclosure, the method may further include statistically analyzing the developed gait speed estimation model.

In the present disclosure, the method may further include calculating a gait speed of the subject for gait speed estimation by using the developed gait speed estimation model.

In the present disclosure, the calculating of the gait speed may include obtaining data regarding an age, a weight, and a foot length of the subject for gait speed estimation by receiving an input, obtaining a cadence, a vertical height displacement (VHD), a roll angle, and a yaw angle from an inertial measurement unit (IMU), and calculating the gait speed by substituting obtained values into the gait speed estimation model.

According to another embodiment of the present disclosure, provided is a device for estimating a gait speed, the device including: a processor; and a memory; wherein the processor is configured to determine N subjects for gait speed measurement and obtaining personal data of the subjects, perform a clinical assessment on the subjects, obtain measured gait data by performing a gait assessment on the subjects, and develop a gait speed estimation model having a plurality of gait factors based on the measured gait data, and the gait speed estimation model includes, as gait factors, a roll angle, a yaw angle, and a body weight measured by using an accelerometer worn on a center of body mass of the subject.

In addition, provided is a computer-readable recording medium having recorded thereon a program for causing a computer to execute the method of the present disclosure.

**Advantageous Effects**

According to the solution of the present disclosure described above, aging and relevant risks may be diagnosed by precisely estimating a gait speed.

In addition, according to the solution of the present disclosure, it is possible to reduce fitting errors by using body weight, roll angle, and yaw angle as additional gait factors, and to develop a gait speed estimation model by using normalized subgroups.

In addition, according to the solution of the present disclosure, a model of the present disclosure may minimize demographic and anthropometric influences on gait speed estimation by using a normalized step length (NSL) instead of a raw step length value.

**Brief Description of Drawings**

FIG. 1 is a system diagram including a user terminal and an estimation server according to an embodiment.
FIG. 2 is a flowchart of a gait speed estimation method according to an embodiment.
FIG. 3 is a table showing characteristics of subjects according to an embodiment.
FIG. 4 is a table showing a comparison between a zeroth model and a first model, according to an embodiment.
FIG. 5 is a table showing subgroups classified by normalized step length (NSL), according to an embodiment.
FIG. 6 is a table showing a gait speed estimation method for each NSL subgroup, according to an embodiment.
FIG. 7 is a comparative example of statistical analysis comparison between gait speed estimation models according to an embodiment, and other models.
FIG. 8 illustrates a gait model of a gait speed estimation model according to an embodiment.
FIG. 9 is a block diagram of a gait speed estimation device according to an embodiment.

**Best Mode**

According to an embodiment, provided is a method of estimating a gait speed, the method including: determining N subjects for gait speed measurement and obtaining personal data of the subjects; obtaining data regarding a result of performing a clinical assessment on the subjects; obtaining measured gait data by performing a gait assessment on the subjects; and developing a gait speed estimation model having a plurality of gait factors based on the measured gait data, wherein the gait speed estimation model includes, as gait factors, a roll angle, a yaw angle, and a body weight measured by using an accelerometer worn on a center of body mass of the subject.

**Mode for Invention**

Advantages and features of the present disclosure and a method for achieving them will be apparent with reference to embodiments of the present disclosure described below together with the accompanying drawings. The present disclosure may, however, be embodied in many different forms and should not be construed as being limited to the embodiments set forth herein, and all changes, equivalents, and substitutes that do not depart from the spirit and technical scope of the present disclosure are encompassed in the present disclosure.

Terms used herein are for describing particular embodiments and are not intended to limit the scope of the present disclosure. Singular forms are intended to include plural forms as well, unless the context clearly indicates otherwise. As used herein, terms such as "comprises," "includes," or "has" specify the presence of stated features, numbers, operations, components, parts, or a combination thereof, but do not preclude the presence or addition of one or more other features, numbers, operations, components, parts, or a combination thereof.

Some embodiments of the present disclosure may be represented by functional block components and various processing operations. Some or all of the functional blocks may be implemented by any number of hardware and/or software elements that perform particular functions. For example, the functional blocks of the present disclosure may be embodied by at least one microprocessor or by circuit components for a certain function. In addition, for example, the functional blocks of the present disclosure may be implemented by using various programming or scripting languages. The functional blocks may be implemented by using various algorithms executable by one or more processors. Furthermore, the present disclosure may employ known technologies for electronic settings, signal processing, and/or data processing. Terms such as "mechanism", "element", "unit", or "component" are used in a broad sense and are not limited to mechanical or physical components.

In addition, connection lines or connection members between components illustrated in the drawings are merely exemplary of functional connections and/or physical or circuit connections. Various alternative or additional functional connections, physical connections, or circuit connections between components may be present in a practical device.

Hereinafter, an operation performed by a user may refer to an operation performed by the user through a user terminal. For example, a command corresponding to an action performed by the user may be input to the user terminal through an input device (e.g., a keyboard or a mouse) embedded in or additionally connected to the user terminal. As another example, a command corresponding to an action performed by the user may be input to the user terminal through a touch screen of the user terminal. Here, the action performed by the user may include a certain gesture. For example, gestures may include tap, touch and hold, double-tap, drag, panning, flick, drag and drop, and the like.

Hereinafter, the present disclosure will be described in detail with reference to the accompanying drawings.

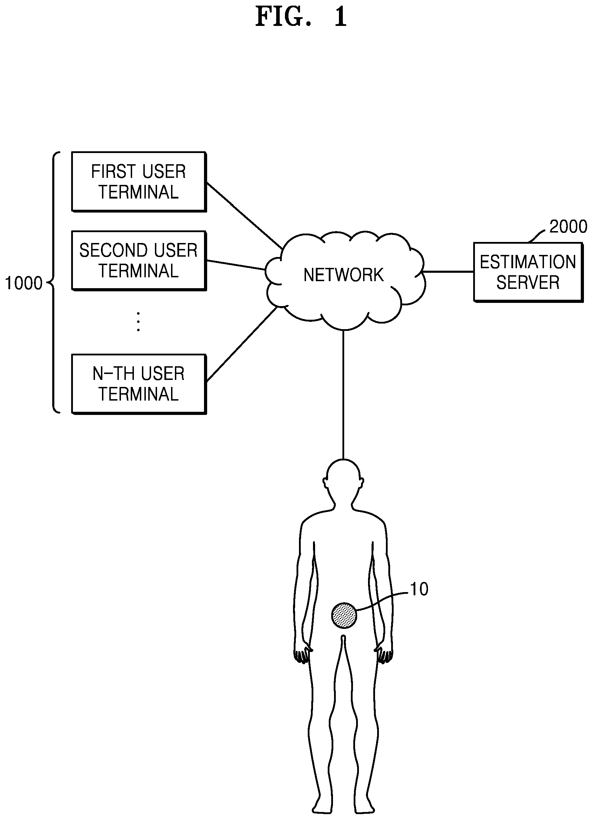

FIG. 1 is a system diagram including a user terminal and an estimation server according to an embodiment.

The system according to an embodiment may include a plurality of user terminals 1000, an estimation server 2000, and an inertial measurement unit (IMU) 10.

The user terminal 1000 may be a smart phone, a tablet personal computer (PC), a PC, a smart television (TV), a mobile phone, a laptop, and other mobile or non-mobile computing devices. In addition, the user terminal 1000 may be a wearable device having a communication function and a data processing function, such as glasses or a hair band. The user terminal 1000 may include all types of devices capable of communicating with other devices through a network.

The estimation server 2000 may be implemented as a computer device or a plurality of computer devices that provide a command, code, a file, content, a service, and the like by performing communication through a network.

The user terminal 1000 and the estimation server 2000 may perform communication by using a network. The estimation server 2000 may communicate with the user terminal 1000 through a network and provide the user terminal 1000 with a gait speed estimation model and an estimation result. In an embodiment, the estimation server 2000 may estimate a gait speed of a user by using a value obtained from the IMU 10.

In addition, in another embodiment, the user terminal 1000 may be a gait speed estimation device that downloads an application for performing a gait speed estimation method of the present disclosure from the estimation server 2000 or another external device (not shown). That is, the gait speed estimation method of the present disclosure may be performed by the user terminal 1000 or the estimation server 2000. Hereinafter, for convenience of description, the user terminal 1000 or the estimation server 2000 will be collectively referred to as a gait speed estimation device.

The IMU 10 is an acceleration measurement device, and more specifically, may be a three-axis acceleration measurement device. According to an embodiment of the present disclosure, as illustrated in FIG. 1, the IMU 10 may be mounted on the center of body mass of a person. In more detail, the IMU 10 may be a three-axis accelerometer attached to the waist by using a certain adhesive material.

In addition, the network may include a local area network (LAN), a wide area network (WAN), a value-added network (VAN), a mobile radio communication network, a satellite communication network, and a combination thereof, may be a comprehensive data communication network that allows each network constituent entity illustrated in FIG. 1 to perform seamless communication with each other, and may include a wired Internet network, a wireless Internet network, and a mobile wireless communication network. In addition, the wireless communication may include, but is not limited to, a wireless LAN (e.g., Wi-Fi), Bluetooth, Bluetooth Low Energy, Zigbee, Wi-Fi Direct (WFD), ultra-wideband (UWB), Infrared Data Association (IrDA), and near-field communication (NFC).

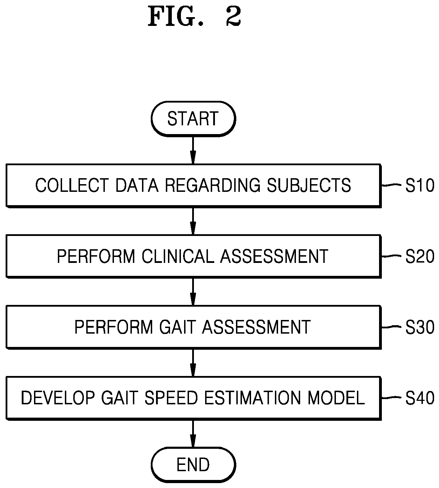

FIG. 2 is a flowchart of a gait speed estimation method according to an embodiment.

Referring to FIG. 2, first, N subjects for gait speed measurement are determined, and personal data of the subjects are collected (S10).

Next, a clinical assessment is performed on the determined subjects (S20).

Next, a gait assessment is performed based on the clinical assessment (S30).

Next, a gait speed estimation model with a plurality of gait factors is developed based on the gait assessment (S40). Here, the gait speed estimation model may include a roll angle, a yaw angle, and a body weight measured by using an accelerometer worn on the center of body mass of the subject as gait factors.

Hereinafter, the gait speed estimation method of the present disclosure will be described in more detail.

First, subjects to be measured in the present disclosure may be specified as follows.

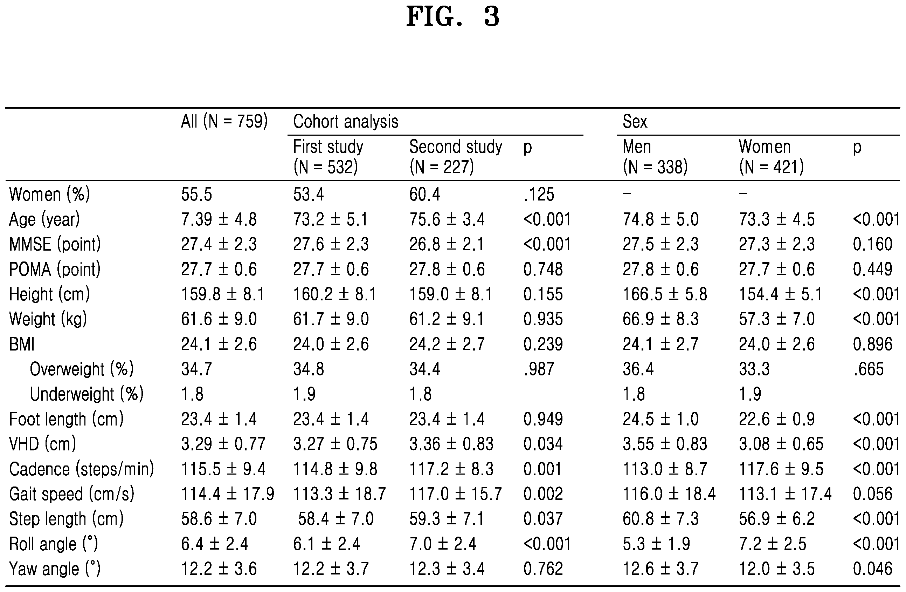

FIG. 3 is a table showing characteristics of subjects according to an embodiment.

As summarized in the table of FIG. 3, the present disclosure may determine, as subjects, some of a total of N participants in two existing cohort studies on aging and frailty, i.e., a first study and a second study. In addition, the present disclosure obtains physical characteristic data of the subjects. For example, the physical characteristic data of the subjects may include sex, age, Mini Mental State Examination (MMSE), Performance Oriented Mobility Assessment (POMA), height, weight, body mass index (BMI) including overweight and underweight proportions, foot length, vertical height displacement (VHD), cadence, gait speed, step length, roll angle, yaw angle, and the like, and may be written in the first column of the table in FIG. 3. In more detail, the VHD may be the mean of differences between the maximum and minimum vertical heights of the center of body mass within one gait cycle. In addition, in the table of FIG. 3, the gait speed and the step length may be obtained by using an existing gait analysis device (e.g., GAITRite™), which will be described later, and the roll angle may be measured by using a waist-worn inertial measurement unit.

In more detail, according to an embodiment of the present disclosure, the first study and the second study may be population-based preliminary cohort studies on elderly Koreans. According to an embodiment, the first study may be "Overview of the Korean longitudinal study on cognitive aging and dementia" (J. W. Han, T. H. Kim, K. P. Kwak, K. Kim, B. J. Kim, S. G. Kim, J. L. Kim, T. H. Kim, S. W. Moon, and J. Y. Park, Psychiatry investigation, vol. 15, no. 8, pp. 767, 2018.), and the second study may be "The Korean version of the FRAIL scale: clinical feasibility and validity of assessing the frailty status of Korean elderly"(H.-W. Jung, H.-J. Yoo, S.-Y. Park, S.-W. Kim, J.-Y. Choi, S.-J. Yoon, C.-H. Kim, and K.-i. Kim, The Korean journal of internal medicine, vol. 31, no. 3, pp. 594, 2016.), but the present disclosure is not limited thereto, and the subjects of the present disclosure may be freely changed.

In more detail, in the first study, elderly people who are sampled from a particular region may be tracked at a preset period. In more detail, in the first study, 6,818 Koreans aged 60 years or older, who were randomly sampled from 13 districts across South Korea, may be tracked every two years from 2009. In the second study, follow-up observation by volunteers may be conducted in a particular region at a preset period. In more detail, in the second study, 3,000 Korean volunteers aged 70 to 84 years may perform follow-up observation every two years from 2016 to 2020.

Meanwhile, from among the M participants in the first and second studies, N community-dwelling healthy elderly people, excluding participants with major mental disorders and neurological disorders such as neurocognitive disorders, may be used as subjects to establish a gait speed estimation model of the present disclosure. That is, according to an embodiment of the present disclosure, people excluding those with mental disorders or neurological disorders from a population consisting of participants of existing studies may be determined as the N subjects.

Referring to the embodiment of FIG. 3, a total of 759 people, including 338 men aged 74.8 ± 5.0 years and 421 women aged 73.3 ± 4.5 years, may be included as subjects. According to an embodiment, participants with stroke and musculoskeletal disorders that may affect gait or balance in a baseline assessment or a follow-up assessment, and participants whose Tinetti POMA scores are less than a preset score (e.g., 25 points) are excluded from the subjects.

Next, according to an embodiment of the present disclosure, a clinical assessment is performed on the determined subjects (S20). In more detail, geriatric psychiatrists or neurologists with expertise in dementia research may perform face-to-face, standardized diagnostic interviews, physical and neurological examinations, and laboratory tests including complete blood counts, chemistry profiles, serological tests for syphilis, echocardiography, and chest X-ray.

In addition, in the present disclosure, dementia and other mental disorders may be diagnosed according to pre-established diagnostic criteria, and the global severity of cognitive disorders may be determined using the clinical dementia rating. In addition, gait and balance may be evaluated by using the POMA described above. In an embodiment, a higher POMA score may indicate better gait and balance, and the maximum POMA score may be 28.

Next, the present disclosure performs a gait assessment to obtain measured gait data (S30). In more detail, the present disclosure may obtain a result of measuring the gait of each subject, by using an IMU attached to the center-of-body-mass (CoM) and an existing gait measurement device. In one example, the IMU may be FITMET®FitLife Inc, but is not limited thereto and may be replaced with ActiGraph®. In addition, in an embodiment, the existing gait measurement device may be GAITRite™ (CI Systems, HAVERT), but is not limited thereto, and a device for generating gait data necessary for development of a gait speed estimation model of the present disclosure, which will be described below, may be any existing gait measurement device without limitation. Hereinafter, for convenience of description, it will be assumed that the existing gait measuring device is GAITRite.

According to an embodiment, the IMU may be a hexagon with smooth edges (35 X 35 X 13 mm [14 g] or 30 X 40 X 10 mm [17 g]), and as an example of the IMU, similar digital three-axis accelerometers (e.g., BMA255, BOSH, Germany) and a gyroscope (e.g. BMX055, BOSH, Germany) may be used. The example three-axis accelerometers may measure three-axis accelerations up to $\pm 8$ g (with a resolution of 0.004 g) and three-axis accelerations up to $\pm 1,000$ μ/s (with a resolution of 0.03 μ/s) at 250 Hz.

In the measurement method according to an embodiment of the present disclosure, the IMU may be fixed to each participant at the third to fourth lumbar vertebrae by a certain adhesive (e.g., Hypafix). In addition, during an experiment, a movement path may be designed such that subjects comfortably walk a 14-m flat straight walkway back and forth three times at a comfortable self-selected pace and turn after passing the 14-m line. In addition, according to an embodiment, a GAITRite electronic mat may be placed in the middle of the walkway to measure steady-state walking.

In an embodiment, GAITRite may be a portable gait analysis walkway system connected to a Universal Serial Bus (USB) port of a computer that measures temporal and spatial gait parameters via an electronic walkway at 100 Hz. The walkway size of the GAITRite electronic mat may be 520 (L) X 90 (W) X 0.6 cm (H), and the active sensing area may be 427 (L) X 61 cm (W). In addition, the electronic mat may include 16,128 sensors arranged with a spatial accuracy of 1.27 cm.

Next, the present disclosure develops a gait speed estimation model (S40). In an embodiment, in order to measure steady-state walking of the subject, the present disclosure may remove preset lengths of the front and rear gait sections from the data generated in the above-described gait data obtaining operation. In more detail, in order to measure steady-state walking of the subject, the gait speed estimation device of the present disclosure may analyze data of the central 10 m of 14-m flat straight walkways remaining after removing 2-m long gait sections before a reference point and before each turn. In addition, the present disclosure may preprocess IMU signals, select features, and identify each step. At this time, the present disclosure may use a comparative standard gait speed obtained from another existing gait measurement device (e.g., GAITRite).

The present disclosure may establish a zeroth model based on the obtained comparative standard gait speed, and estimate a gait speed by using IMU signals according to the zeroth model. In the zeroth model, a gait speed may be estimated with a regression model including age, sex, foot length, cadence, VHD, and cadence (the number of steps per minute).

In more detail, according to an embodiment, the zeroth model may be a regression model for estimating a gait speed of a healthy elderly person by using the acceleration and angle of an IMU sensor. In the zeroth model, two demographic characteristics (age and sex), an anthropometric characteristic (foot length), and two characteristics measured by an IMU (VHD and cadence) may be used as gait factors to estimate a gait speed.

Meanwhile, a gait speed is the product of a cadence and a step length. Regarding gait speed estimation, a cadence measured by an IMU is reliable, and thus, including characteristics associated with step length in the gait speed estimation model may improve the accuracy of the model.

[Equation 1] below is a mathematical linear regression equation of the zeroth model developed based on the personal data of the subjects obtained in the embodiment of FIG. 2 described above. From [Equation 1], it may be seen that the linear regression equation of the zeroth model includes age, sex, cadence, VHD, and foot length as gait factors for estimating a gait speed, and the linear regression equation may have a coefficient corresponding to each gait factor. In addition, a foot length is the mean length of both feet, and sex may be set to 1 for male and 2 for female.

[Equation 1]

*Gait speed [model 0]*

*= -113.2-0.388\*Age+3.06\*Sex+1.17\*Cadence+12.1\*VHD+3.25\*Foot length*

However, there was a problem in that the zeroth model showed low accuracy for people whose gait speed was less than 100 cm per second, and the estimation error gradually increased as the speed decreased. Accordingly, in the present disclosure, the zeroth model for estimate a gait speed may be supplemented by adding characteristics associated with step length, and the gait speed estimation model of the present disclosure may be optimized within subgroups stratified by normalized step length (NSL). In addition, elderly people may have different gait patterns depending on their health status, however, in order to compensate for the vulnerability of the linear regression model to nonhomogeneous samples, the accuracy of the model may be further improved by developing a regression model within sequential subgroups of gait speed. In addition, the accuracy of the gait speed estimation model of the present disclosure was compared with the accuracy of other existing gait analysis systems.

Hereinafter, as an embodiment of the present disclosure, a first model and a second model of the gait speed estimation method that may complement the above-described zeroth model will be described.

According to an embodiment of the present disclosure, the first model of the gait speed estimation method of the present disclosure may be developed by adding roll and yaw angles of the center of body mass and a body weight as gait factors to the zeroth model.

According to an embodiment, a step length is a product of motions of torso, knees, and feet, and roll and yaw angles of the center of body mass may reflect angular motions of the torso and limbs. In more detail, the roll and yaw angles of the center of body mass may be calculated from sensor data of an IMU, and roll and yaw angle values may be reoriented to Cartesian coordinates. The step length is also associated with the body weight that is associated with thrust power.

In more detail, three additional characteristics selected in the linear regression model of the first model of the present disclosure are a body weight, and a roll angle and a yaw angle of the CoM. In regard to this, the body weight is a major gait factor in that both weight loss and gain reduce the gait speed by reducing muscle power or inducing physical frailty. In addition, in older adult samples, a BMI of 25 or greater, which is considered overweight, is more common than a BMI less than 18.5, which is considered underweight, and thus, this is the reason why the body weight is added as a gait factor in the gait speed estimation model of the present disclosure, and why the coefficients are negative in the regression model.

In addition, body rotation is associated with gait patterns. A roll angle and a yaw angle represent a three-dimensional gait pattern. Viewed from the top of the body, a yaw rotation adds a CoM motion to a forward ground motion of the stance leg when the subject rotates the waist in a gait in which each leg moves forward. In the gait speed estimation model of the present disclosure, the yaw angle coefficient is positive, which may indicate that the yaw angle may increase as the gait speed increases. As such, the present disclosure may define the relationship between the yaw angle of the CoM and the gait speed, which has not been studied previously.

In addition, a roll angle represents a motion associated with medio-lateral (ML) stability and is associated with the gait speed. In the gait speed estimation model of the present disclosure, the roll angle coefficient is positive, which indicates that the roll angle may increase as the gait speed increases.

Meanwhile, sex may not be selected as a gait factor in the first model and the second model of the gait speed estimation model of the present disclosure. This is because, among the three functions added to the model of the present disclosure, the roll angle of the CoM may reflect the influence of sex on the gait speed to some extent. That is, as may be seen in the table of FIG. 3 described above, women have a larger roll angle than men. On the contrary, the yaw angle is not statistically different between the sexes.

[Equation 2] below is a mathematical linear regression equation of the first model developed based on the personal data of the subjects obtained in the embodiment of FIG. 2 described above. From [Equation 2], it may be seen that the linear regression equation of the first model includes age, cadence, VHD, foot length, body weight, roll angle, and yaw angle as gait factors for estimating a gait speed, and the linear regression equation may have a coefficient corresponding to each gait factor.

[Equation 2]

*Gait speed [model 1]*

$= -106.0-0.328*Age+1.10*Cadence+10.1*VHD+3.29*Foot\ length-0.115*Weight+1.01*Roll\ angle+0.647*Yaw\ angle$

According to an embodiment of the present disclosure, a gait speed of a subject for gait speed estimation may be accurately calculated by using the developed first model. That is, the gait speed estimation device of the present disclosure may obtain data regarding the age, weight, and foot length of a subject whose gait speed is to be calculated, by receiving an input, or may obtain the data from another terminal or server. In addition, the gait speed estimation device obtains, from an IMU, a cadence, a VHD, a roll angle, and a yaw angle, which are values measured by the IMU while the subject for gait speed estimation wearing the IMU walks. In addition, the gait speed estimation device may calculate the gait speed by substituting the obtained values of the gait factors into the linear regression equation of the first model. According to an embodiment of the present disclosure, when the gait speed is estimated by using the developed first model, precise estimation of the gait speed may be possible by simply using an IMU even without a gait measurement device (e.g., GAITRite).

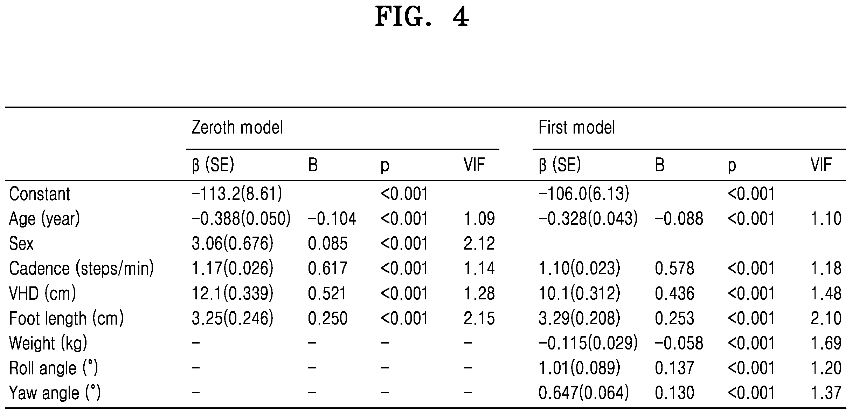

FIG. 4 is a table showing a comparison between a zeroth model and a first model, according to an embodiment.

Referring to FIG. 4, the first model of the present disclosure may include sex, roll angle, yaw angle, and body weight as gait factors, and gait factors of the zeroth model may be used as the other gait factors. In the table of FIG. 4, VIF denotes variation inflation factor.

In addition, it may be seen that the zeroth model estimates a gait speed quite well (R = 0.935, $R^2$ = 0.875, adjust $R^2$ = 0.874, F = 1051, p < 0.001) when compared to a value measured by Gaitrite, but a gait speed estimated by the first model is more accurate (R = 0.953, $R^2$ = 0.908, adjust$R^2$ = 0.908, F = 10.001, p < 0.001).

In addition, the present disclosure may use k-means clustering to set a second model, which is a regression model for each subgroup with the gait factors included in the first model described above, within subgroups classified by NSL. In the regression model of the second model of the present disclosure, only characteristics with a VIF of less than 2.5 may be included.

According to an embodiment of the present disclosure, the NSL may be obtained by dividing the step length by the height, and the step length may be obtained by dividing the gait speed obtained from the first model by the cadence. Hereinafter, a combination of the above-described first model and second model may be referred to as the gait speed estimation model of the present disclosure.

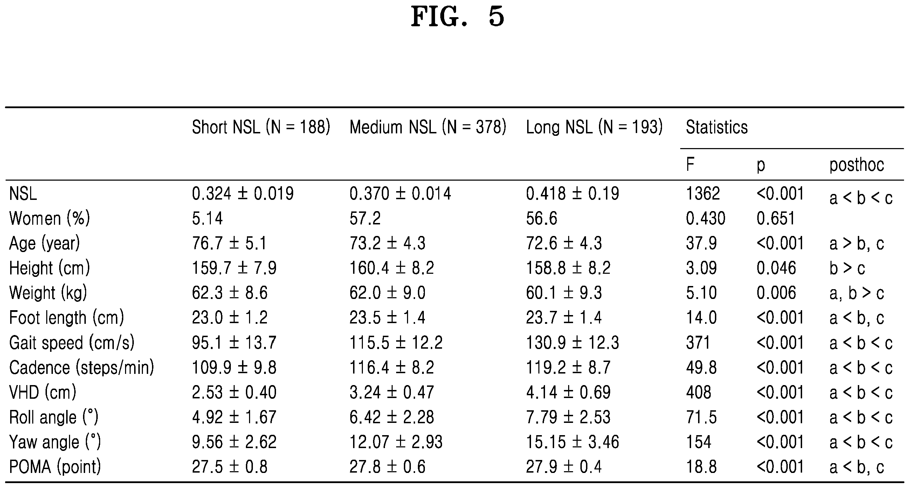

FIG. 5 is a table showing subgroups classified by NSL, according to an embodiment.

Referring to FIG. 5, characteristics of three NSL subgroups are summarized in the table of FIG. 5. In more detail, the NSL subgroups may include short NSL, medium NSL, and long NSL. According to an embodiment of the present disclosure, reference values for distinguishing between the short NSL, medium NSL, and long NSL subgroups may be set differently depending on the characteristics of the subjects, and the number of subgroups may also be set to be more or less than three. FIG. 5 shows physical characteristic data of subjects of respective subgroups classified based on preset reference values, according to an embodiment of the present disclosure.

In more detail, referring to FIG. 5, the present disclosure may obtain sex, age, height, weight, foot length, gait speed, cadence, VHD, roll angle, yaw angle, and POMA values of the subjects for each subgroup. The VHD and the POMA are the same as described above with reference to FIG. 3, the roll angle and the yaw angle may be obtained from an IMU, and the foot length and the gait speed may also be obtained from GAITRite as described above with reference to FIG. 3. Descriptions of the other values that are provided above with reference to FIG. 3 will be omitted. In addition, according to an embodiment, the statistics in FIG. 3 use, as statistical analysis, analysis of variance based on Bonferroni post hoc comparison, but obviously, the statistical analysis method may be changed in other embodiments.

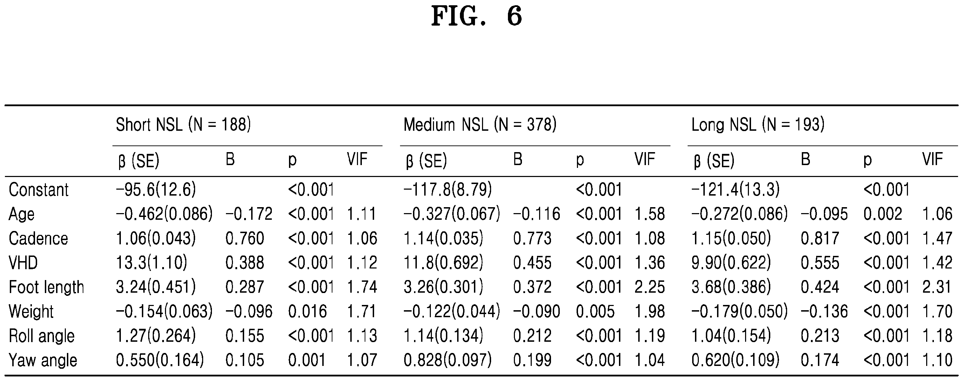

FIG. 6 is a table showing a gait speed estimation method for each NSL subgroup, according to an embodiment.

Referring to FIG. 6, it may be seen that, when the second model, which is a gait speed estimation model for each NSL subgroup, was developed by using the gait factors included in the first model, all gait factors were selected as significant gait factors for gait speed in all NSL subgroups and have low VIF.

Referring to FIG. 6, the model for each NSL subgroup (the second model) according to an embodiment may output a more accurately estimated gait speed (R = 0.955, $R^2$ = 0.912, p < 0.001) than the zeroth model and the first model, and the gait speed estimates may be appropriate for all NSL subgroups. In more detail, for the NSL subgroups, statistical values of gait speed estimated by the second model may be $R^2$ = 0.828, F=195.545, p < 0.001 for the short NSL subgroup (Short NSL in FIG. 6), $R^2$ = 0.801, F = 217.135, p < 0.001 for the medium NSL subgroup(Medium NSL in FIG. 6), and R2 = 0.836, F = 140.971, p < 0.001 for the long NSL subgroup (Long NSL in FIG. 6).

In addition, [Equation 3-1] to [Equation 3-3] below are mathematical linear regression equations of the second models shown in FIG. 6, which are developed based on the personal data of the subjects obtained in the embodiment of FIG. 2 described above. It may be seen from [Equation 3-1] to [Equation 3-3] that the linear regression equation for each NSL subgroup of the second model includes age, cadence, VHD, foot length, body weight, roll angle, and yaw angle as gait factors for estimating a gait speed, and may have a coefficient corresponding to each gait factor. In addition, the short NSL subgroup may be set as a group with an NSL of 0.347 or less, the medium NSL subgroup may be set as a group with an NSL of greater than 0.347 but less than or equal to 0.394, and the long NSL subgroup may be set as a group with an NSL of greater than 0.394.

[Equation 3-1]

*Gait speed [model 2, NSL≤0.347]*

*=        -95.6-0.462\*Age+1.06\*Cadence+13.3\*VHD+3.24\*Foot        length-0.154\*Weight+1.27\*Roll angle+0.550\*Yaw angle*

[Equation 3-2]

*Gait speed [model 2, 0.347<NSL≤0.394]*

*=          -117.8-0.327\*Age+1.14\*Cadence+11.8\*VHD+3.26\*Foot          length-0.122\*Weight+1.14\*Roll angle+0.828\*Yaw angle*

[Equation 3-3]

*Gait speed [model 2, 0.394<NSL]*

*=          -121.4-0.272\*Age+1.15\*Cadence+9.90\*VHD+3.68\*Foot          length-0.179\*Weight+1.04\*Roll angle+0.620\*Yaw angle*

According to an embodiment of the present disclosure, a gait speed of a subject for gait speed estimation may be accurately calculated for each NSL subgroup by using the developed second model. That is, the gait speed estimation device of the present disclosure may obtain data regarding the age, weight, and foot length of a subject whose gait speed is to be calculated, by receiving an input, or may obtain the data from another terminal or server. In addition, the gait speed estimation device obtains, from an IMU, a cadence, a VHD, a roll angle, and a yaw angle, which are values measured by the IMU while the subject for gait speed estimation wearing the IMU walks. The gait speed estimation device may also use the values of the gait factors obtained in the using of the first model described above, for the second model.

In addition, the gait speed estimation device may use a gait speed calculated from the first model to determine the NSL subgroup of the subject for gait speed estimation. Here, the NSL of the subject for gait speed estimation may be calculated according to *NSL= (gait speed of model 1)/(candence/60)/height,* and the NSL subgroup may be determined by using the calculated NSL. In addition, the gait speed estimation device may calculate a gait speed by substituting the obtained values of the gait factors into the linear regression equation of the second model (the regression equation of the corresponding NSL subgroup). According to an embodiment of the present disclosure, when the gait speed is estimated by using the developed second model, precise estimation of the gait speed for each NSL subgroup may be possible by simply using an IMU even without a gait measurement device (e.g., GAITRite).

Next, the present disclosure may perform statistical analysis on the developed gait speed estimation model. According to an embodiment, the present disclosure may compare continuous variables by using a Student's t-test or analysis of variance (ANOVA), and compare categorical variables by using a chi-squared test between groups. By using such a statistical analysis method, the present disclosure may compare statistical values estimated by Gaitrite with statistical values estimated by the gait speed estimation model of the present disclosure. In more detail, the statistical values to be compared may be intraclass correlation coefficient (ICC), mean error (ME), mean absolute error (MAE), and root mean square error (RMSE).

In addition, the present disclosure may compare the ICC, ME, MAE, and RMSE of estimated gait speeds between gait speed estimation models by using repeated measures ANOVA. The present disclosure may perform all statistical analyses by using the Statistical Package for the Social Sciences version 25.0 (International Business Machines Corporation, Armonk, NY). In an embodiment of the present disclosure, statistical analysis may be performed by setting the zeroth model of the gait speed estimation model of the present disclosure as an existing technique, and setting the first model and the second model as developed techniques.

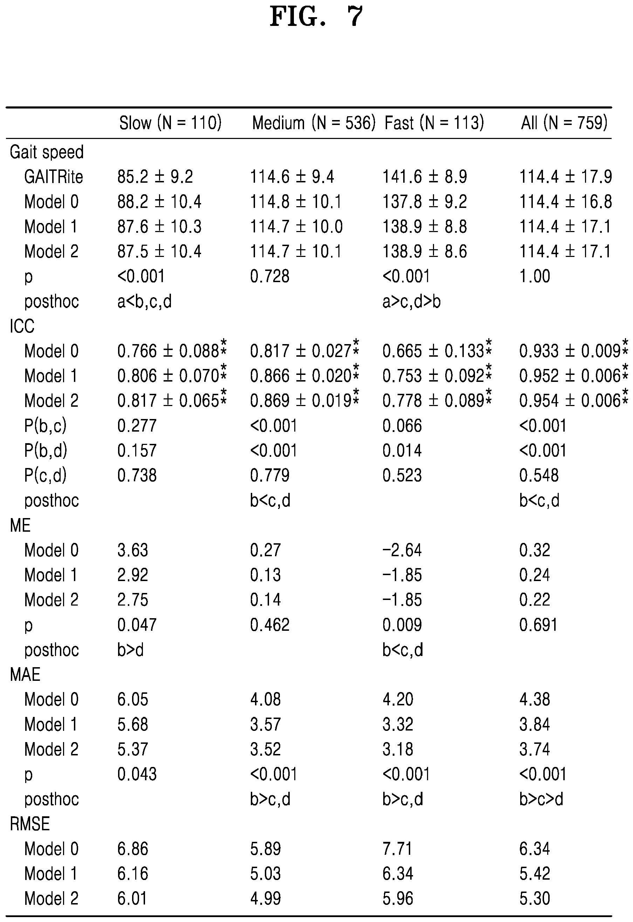

FIG. 7 is a comparative example of statistical analysis comparison between gait speed estimation models according to an embodiment, and other models.

Referring to FIG. 7, the present disclosure may compare the accuracy between models of the gait speed estimation method of the present disclosure by using a gait speed measured by GAITRite as a comparison standard. Referring to FIG. 7, it may be seen that for all subjects, the first model and the second model show lower MAEs, MEs, and RMSEs and better ICCs than the zeroth model. In addition, it may be seen that the second model shows a lower MAE than the first model (p = 0.007).

In addition, referring to FIG. 7, according to the second model of the present disclosure, the subjects may be grouped into three gait subgroups (Slow, Medium, and Fast) by using gait speeds based on GAITRite. Slow gait was defined as a standard deviation of the mean (17.9 m/s) or less, and fast gait was defined as a standard deviation of the mean (114.4 m/s) or greater.

Gait speeds estimated by the zeroth model tended to be slower than that measured by GAITRite in the fast NSL subgroup (p < 0.001), but faster in the slow NSL subgroup (p < 0.001). This is similar for gait speeds estimated by the first model and the second model, but it may be seen that the difference in gait speed measurements between the IMU and GAITRite™ decreased in the fast NSL subgroup (p < 0.001).

In summary, gait speed estimation by linear regression according to an embodiment of the present disclosure is essentially intended to produce an approximation of the mathematical expression of human gait speed. In the model of the present disclosure, linear regression may be a simplified from of Taylor expansion of a virtual true expression, and may include higher degrees and number of variables. In addition, the present disclosure may improve the accuracy of gait speed estimation using an IMU by using additional gait factors in the linear regression model and optimizing the linear regression model in the NSL subgroups.

In addition, the gait speed estimation model of the present disclosure may use NSL to develop a model for each subgroup. The step length decreases with age, and thus may reflect gait patterns of elderly people. As shown in FIG. 5 described above, anthropometric measurements differed between the three NSL subgroups, and the three subgroups may have different gait patterns associated with different anthropometric characteristics.

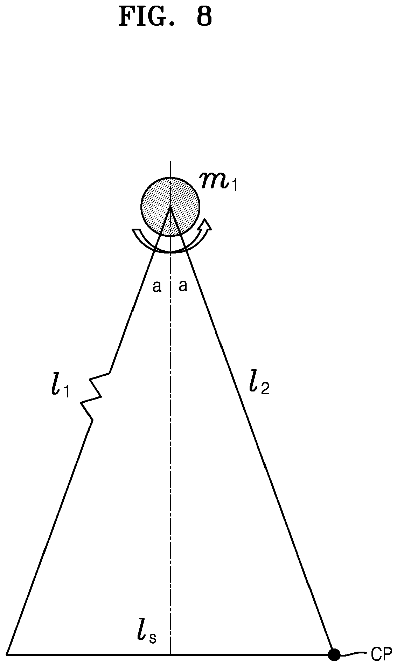

FIG. 8 illustrates a gait model of a gait speed estimation model according to an embodiment.

The model of the present disclosure may minimize demographic and anthropometric influences on gait speed estimation by using an NSL instead of a raw step length value. A human gait during a stance phase may be modeled as a spring-loaded inverted pendulum as illustrated in FIG. 8. Here, CP denotes a contact point. The leg length in the stance phase may be expressed as [Equation 4] below. Here, $l_1$ denotes the leg length and $\beta_1$ denotes the coefficient of leg bending.

[Equation 4]

$$l_l = \beta_1(t) l_1$$

In addition, a step length may be calculated. Considering the conservation of angular momentum and point mass assumption, energy conserved $E_{con}$ after contact may be expressed as [Equation 5] below. Here, mi denotes the point mass, $\omega_1$ denotes the angular velocity before contact, $l_1$ denotes the leg length, and $l_s$ denotes the step length.

[Equation 5]

$$E_{con} = \frac{1}{2} m_1 l_l^2 \omega_1^2 [1 - (\frac{l_s^2}{4 l_1^2})^2]$$

Thus, considering the symmetry of motion, the step length may be calculated as in [Equation 6] below.

[Equation 6]

$$l_s \approx 2 l_1 \beta_1(0) \sin(\alpha)$$

Thus, the NSL may be calculated as in [Equation 7] below. Here, $\alpha_1$ denotes the human leg/height ratio, and a denotes the angular displacement in gait mechanics.

[Equation 7]

$$NSL = \frac{l_s}{h} \approx \frac{2 l_1}{h} \beta_1(0) \sin(a) = 2 \alpha_1 \beta_1(0) \sin(a)$$

In the first model of the present disclosure, the VHD coefficients are different among the three NSL subgroups. In more detail, in the second model, the VHD coefficient was highest in the short NSL subgroup and lowest in the long NSL subgroup, whereas B was smallest in the short NSL subgroup, and thus, it may be seen that the short NSL subgroup has less effective dynamic gait and body rotation than the other NSL subgroups. In addition, the coefficient of age and B value were lowest in the long NSL subgroup, suggesting that the gait of the long NSL subgroup may be less affected by age. In addition, the cadence was low in the short NSL subgroup, indicating that the short NSL subgroup may have a more conservative gait pattern than other subgroups.

In summary, the present disclosure generated a gait speed estimation model based on results of analyzing gait patterns of N elderly people. The present disclosure may improve existing speed estimation models by using additional gait factors to reduce fitting errors and allow further development of estimation equations by using normalized subgroups.

According to an embodiment of the present disclosure, the additional gait factors are roll angle, yaw angle and weight, which are associated with motions below the center of body mass in terms of angle and propulsion. By adding additional gait factors, a better estimation result may be derived than sex-based estimation in existing studies. In addition, because the NSL subgroups are generated by dividing the subjects with different gait mechanics regardless of size, the gait speed estimation algorithm may be improved with high accuracy.

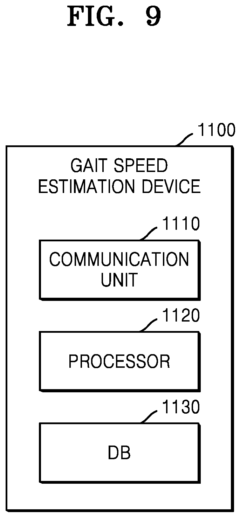

FIG. 9 is a block diagram of a gait speed estimation device according to an embodiment.

A gait speed estimation device 1100 of FIG. 9 may be the gait speed estimation device of FIG. 1.

Referring to FIG. 9, the gait speed estimation device 1100 may include a communication unit 1110, a processor 1120, and a database (DB) 1130. In the gait speed estimation device 1100 of FIG. 9, only components related to the embodiment are illustrated. Therefore, it would be understood by those of skill in the art that other general-purpose components may be further included in addition to those illustrated in FIG. 9.

The communication unit 1110 may include one or more components for performing wired/wireless communication with other nodes. For example, the communication unit 1110 may include at least one of a short-range communication unit (not shown), a mobile communication unit (not shown), and a broadcast receiver (not shown).

The DB 1130 is hardware for storing various pieces of data processed by the gait speed estimation device 1100, and may store a program for the processor 1120 to perform processing and control. The DB 1130 may store payment information, user information, and the like.

The DB 1130 may include random-access memory (RAM) such as dynamic RAM (DRAM) or static RAM (SRAM), read-only memory (ROM), electrically erasable programmable ROM (EEPROM), a compact disc-ROM (CD-ROM), a Blu-ray or other optical disk storage, a hard disk drive (HDD), a solid-state drive (SSD), or flash memory.

The processor 1120 controls the overall operation of the gait speed estimation device 1100. For example, the processor 1120 may execute programs stored in the DB 1130 to control the overall operation of an input unit (not shown), a display (not shown), the communication unit 1110, the DB 1130, and the like. The processor 1120 may execute programs stored in the DB 1130 to control the operation of the gait speed estimation device 1100.

The processor 1120 may be implemented by using at least one of application-specific integrated circuits (ASICs), digital signal processors (DSPs), digital signal processing devices (DSPDs), programmable logic devices (PLDs), field-programmable gate arrays (FPGAs), controllers, microcontrollers, microprocessors, and other electrical units for performing functions.

An embodiment of the present disclosure may be implemented as a computer program that may be executed through various components on a computer, and such a computer program may be recorded in a computer-readable medium. In this case, the medium may include a magnetic medium, such as a hard disk, a floppy disk, or a magnetic tape, an optical recording medium, such as a CD-ROM or a digital video disc (DVD), a magneto-optical medium, such as a floptical disk, and a hardware device specially configured to store and execute program instructions, such as ROM, RAM, or flash memory.

Meanwhile, the computer program may be specially designed and configured for the present disclosure or may be well-known to and usable by those skilled in the art of computer software. Examples of the computer program may include not only machine code, such as code made by a compiler, but also high-level language code that is executable by a computer by using an interpreter or the like.

According to an embodiment, the method according to various embodiments of the present disclosure may be included in a computer program product and provided. The computer program product may be traded as commodities between sellers and buyers. The computer program product may be distributed in the form of a machine-readable storage medium (e.g., a CD-ROM), or may be distributed online (e.g., downloaded or uploaded) through an application store (e.g., Play Store™) or directly between two user devices. In a case of online distribution, at least a portion of the computer program product may be temporarily stored in a machine-readable storage medium such as a manufacturer's server, an application store's server, or a memory of a relay server.

**Claims**

**1.** A method of estimating a gait speed, the method comprising:

determining N subjects for gait speed measurement and obtaining personal data of the subjects;
obtaining data regarding a result of performing a clinical assessment on the subjects;
obtaining measured gait data by performing a gait assessment on the subjects; and
developing a gait speed estimation model having a plurality of gait factors based on the measured gait data,

wherein the gait speed estimation model comprises, as gait factors, a roll angle, a yaw angle, and a body weight measured by using an accelerometer worn on a center of body mass of each of the subjects.

2. The method of claim 1, wherein the determining of the N subjects comprises determining, as the N subjects, people in a population after excluding those who have diseases that is likely to affect a gait or whose scores of a performance-oriented mobility assessment are less than a preset score.

3. The method of claim 1, wherein the clinical assessment is to diagnose mental disorders according to pre-established diagnostic criteria, determine global severity of cognitive disorders, and evaluate gait and balance abilities by using Performance Oriented Mobility Assessment (POMA).

4. The method of claim 1, wherein the obtaining of the measured gait data comprises obtaining gait characteristic values of each of the subjects by using the accelerometer attached to the center of body weight and an existing gait measurement device.

5. The method of claim 1, wherein the accelerometer is an inertial measurement unit (IMU) attached to waist of each of the subjects with a predetermined adhesive material, and is a device capable of measuring three-axis acceleration.

6. The method of claim 1, wherein the developing of the gait speed estimation model comprises:

   a first model that performs linear regression analysis comprising roll angle, yaw angle, and body weight as gait factors; and
   a second model that classifies the subjects into a plurality of subgroups based on normalized step length (NSL), and performs the linear
   regression analysis for each subgroup.

7. The method of claim 6, wherein the NSL is calculated according to an equation:

$$NSL = \frac{l_s}{h} \approx \frac{2l_1}{h}\beta_1(0)\sin(a) = 2\alpha_1\beta_1(0)\sin(a)$$
,

   and
   in the above equation, $l_1$ denotes a leg length, $l_s$ denotes a step length, $\alpha_1$ denotes a human leg/height ratio, $\beta_1$ denotes a coefficient of leg bending, and a denotes an angular displacement.

8. The method of claim 1, further comprising statistically analyzing the gait speed estimation model which is developed.

9. The method of claim 1, further comprising calculating a gait speed of each of the subjects for gait speed estimation by using the gait speed estimation model which is developed.

10. The method of claim 9, wherein the calculating of the gait speed comprises obtaining data regarding an age, a weight, and a foot length of each of the subjects for gait speed estimation by receiving an input, obtaining a cadence, a vertical height displacement (VHD), a roll angle, and a yaw angle from an inertial measurement unit (IMU), and calculating the gait speed by substituting obtained values into the gait speed estimation model.

11. A device for estimating a gait speed, the device comprising:

   a processor; and
   a memory;
   wherein the processor is configured to determine N subjects for gait speed measurement and obtaining personal data of the subjects, perform a clinical assessment on the subjects, obtain measured gait data by performing a gait assessment on the subjects, and develop a gait speed estimation model having a plurality of gait factors based on the measured gait data, and
   the gait speed estimation model comprises, as gait factors, a roll angle, a yaw angle, and a body weight measured by using an accelerometer worn on a center of body mass of each of the subjects.

**12.** A computer-readable recording medium having recorded thereon a program for causing a computer to execute the method of claim 1.

# FIG. 1

# FIG. 2

```
        ┌─────────┐
        │  START  │
        └─────────┘
             │
             ▼
┌──────────────────────────────────────┐
│   COLLECT DATA REGARDING SUBJECTS     │──── S10
└──────────────────────────────────────┘
             │
             ▼
┌──────────────────────────────────────┐
│      PERFORM CLINICAL ASSESSMENT      │──── S20
└──────────────────────────────────────┘
             │
             ▼
┌──────────────────────────────────────┐
│        PERFORM GAIT ASSESSMENT        │──── S30
└──────────────────────────────────────┘
             │
             ▼
┌──────────────────────────────────────┐
│  DEVELOP GAIT SPEED ESTIMATION MODEL  │──── S40
└──────────────────────────────────────┘
             │
             ▼
        ┌─────────┐
        │   END   │
        └─────────┘
```

# FIG. 3

| | All (N = 759) | Cohort analysis | | | Sex | | |
|---|---|---|---|---|---|---|---|
| | | First study (N = 532) | Second study (N = 227) | p | Men (N = 338) | Women (N = 421) | p |
| Women (%) | 55.5 | 53.4 | 60.4 | .125 | – | – | |
| Age (year) | 7.39 ± 4.8 | 73.2 ± 5.1 | 75.6 ± 3.4 | <0.001 | 74.8 ± 5.0 | 73.3 ± 4.5 | <0.001 |
| MMSE (point) | 27.4 ± 2.3 | 27.6 ± 2.3 | 26.8 ± 2.1 | <0.001 | 27.5 ± 2.3 | 27.3 ± 2.3 | 0.160 |
| POMA (point) | 27.7 ± 0.6 | 27.7 ± 0.6 | 27.8 ± 0.6 | 0.748 | 27.8 ± 0.6 | 27.7 ± 0.6 | 0.449 |
| Height (cm) | 159.8 ± 8.1 | 160.2 ± 8.1 | 159.0 ± 8.1 | 0.155 | 166.5 ± 5.8 | 154.4 ± 5.1 | <0.001 |
| Weight (kg) | 61.6 ± 9.0 | 61.7 ± 9.0 | 61.2 ± 9.1 | 0.935 | 66.9 ± 8.3 | 57.3 ± 7.0 | <0.001 |
| BMI | 24.1 ± 2.6 | 24.0 ± 2.6 | 24.2 ± 2.7 | 0.239 | 24.1 ± 2.7 | 24.0 ± 2.6 | 0.896 |
| Overweight (%) | 34.7 | 34.8 | 34.4 | .987 | 36.4 | 33.3 | .665 |
| Underweight (%) | 1.8 | 1.9 | 1.8 | | 1.8 | 1.9 | |
| Foot length (cm) | 23.4 ± 1.4 | 23.4 ± 1.4 | 23.4 ± 1.4 | 0.949 | 24.5 ± 1.0 | 22.6 ± 0.9 | <0.001 |
| VHD (cm) | 3.29 ± 0.77 | 3.27 ± 0.75 | 3.36 ± 0.83 | 0.034 | 3.55 ± 0.83 | 3.08 ± 0.65 | <0.001 |
| Cadence (steps/min) | 115.5 ± 9.4 | 114.8 ± 9.8 | 117.2 ± 8.3 | 0.001 | 113.0 ± 8.7 | 117.6 ± 9.5 | <0.001 |
| Gait speed (cm/s) | 114.4 ± 17.9 | 113.3 ± 18.7 | 117.0 ± 15.7 | 0.002 | 116.0 ± 18.4 | 113.1 ± 17.4 | 0.056 |
| Step length (cm) | 58.6 ± 7.0 | 58.4 ± 7.0 | 59.3 ± 7.1 | 0.037 | 60.8 ± 7.3 | 56.9 ± 6.2 | <0.001 |
| Roll angle (°) | 6.4 ± 2.4 | 6.1 ± 2.4 | 7.0 ± 2.4 | <0.001 | 5.3 ± 1.9 | 7.2 ± 2.5 | <0.001 |
| Yaw angle (°) | 12.2 ± 3.6 | 12.2 ± 3.7 | 12.3 ± 3.4 | 0.762 | 12.6 ± 3.7 | 12.0 ± 3.5 | 0.046 |

EP 4 458 266 A1

# FIG. 4

| | Zeroth model | | | | First model | | | |
|---|---|---|---|---|---|---|---|---|
| | β (SE) | B | p | VIF | β (SE) | B | p | VIF |
| Constant | −113.2(8.61) | | <0.001 | | −106.0(6.13) | | <0.001 | |
| Age (year) | −0.388(0.050) | −0.104 | <0.001 | 1.09 | −0.328(0.043) | −0.088 | <0.001 | 1.10 |
| Sex | 3.06(0.676) | 0.085 | <0.001 | 2.12 | | | | |
| Cadence (steps/min) | 1.17(0.026) | 0.617 | <0.001 | 1.14 | 1.10(0.023) | 0.578 | <0.001 | 1.18 |
| VHD (cm) | 12.1(0.339) | 0.521 | <0.001 | 1.28 | 10.1(0.312) | 0.436 | <0.001 | 1.48 |
| Foot length (cm) | 3.25(0.246) | 0.250 | <0.001 | 2.15 | 3.29(0.208) | 0.253 | <0.001 | 2.10 |
| Weight (kg) | – | – | – | – | −0.115(0.029) | −0.058 | <0.001 | 1.69 |
| Roll angle (°) | – | – | – | – | 1.01(0.089) | 0.137 | <0.001 | 1.20 |
| Yaw angle (°) | – | – | – | – | 0.647(0.064) | 0.130 | <0.001 | 1.37 |

EP 4 458 266 A1

## FIG. 5

| | Short NSL (N = 188) | Medium NSL (N = 378) | Long NSL (N = 193) | Statistics | | |
| --- | --- | --- | --- | --- | --- | --- |
| | | | | F | p | posthoc |
| NSL | 0.324 ± 0.019 | 0.370 ± 0.014 | 0.418 ± 0.19 | 1362 | <0.001 | a < b < c |
| Women (%) | 5.14 | 57.2 | 56.6 | 0.430 | 0.651 | |
| Age (year) | 76.7 ± 5.1 | 73.2 ± 4.3 | 72.6 ± 4.3 | 37.9 | <0.001 | a > b, c |
| Height (cm) | 159.7 ± 7.9 | 160.4 ± 8.2 | 158.8 ± 8.2 | 3.09 | 0.046 | b > c |
| Weight (kg) | 62.3 ± 8.6 | 62.0 ± 9.0 | 60.1 ± 9.3 | 5.10 | 0.006 | a, b > c |
| Foot length (cm) | 23.0 ± 1.2 | 23.5 ± 1.4 | 23.7 ± 1.4 | 14.0 | <0.001 | a < b, c |
| Gait speed (cm/s) | 95.1 ± 13.7 | 115.5 ± 12.2 | 130.9 ± 12.3 | 371 | <0.001 | a < b < c |
| Cadence (steps/min) | 109.9 ± 9.8 | 116.4 ± 8.2 | 119.2 ± 8.7 | 49.8 | <0.001 | a < b < c |
| VHD (cm) | 2.53 ± 0.40 | 3.24 ± 0.47 | 4.14 ± 0.69 | 408 | <0.001 | a < b < c |
| Roll angle (°) | 4.92 ± 1.67 | 6.42 ± 2.28 | 7.79 ± 2.53 | 71.5 | <0.001 | a < b < c |
| Yaw angle (°) | 9.56 ± 2.62 | 12.07 ± 2.93 | 15.15 ± 3.46 | 154 | <0.001 | a < b < c |
| POMA (point) | 27.5 ± 0.8 | 27.8 ± 0.6 | 27.9 ± 0.4 | 18.8 | <0.001 | a < b, c |

EP 4 458 266 A1

# FIG. 6

| | Short NSL (N = 188) | | | | Medium NSL (N = 378) | | | | Long NSL (N = 193) | | | |
|---|---|---|---|---|---|---|---|---|---|---|---|---|
| | β (SE) | B | p | VIF | β (SE) | B | p | VIF | β (SE) | B | p | VIF |
| Constant | −95.6(12.6) | | <0.001 | | −117.8(8.79) | | <0.001 | | −121.4(13.3) | | <0.001 | |
| Age | −0.462(0.086) | −0.172 | <0.001 | 1.11 | −0.327(0.067) | −0.116 | <0.001 | 1.58 | −0.272(0.086) | −0.095 | 0.002 | 1.06 |
| Cadence | 1.06(0.043) | 0.760 | <0.001 | 1.06 | 1.14(0.035) | 0.773 | <0.001 | 1.08 | 1.15(0.050) | 0.817 | <0.001 | 1.47 |
| VHD | 13.3(1.10) | 0.388 | <0.001 | 1.12 | 11.8(0.692) | 0.455 | <0.001 | 1.36 | 9.90(0.622) | 0.555 | <0.001 | 1.42 |
| Foot length | 3.24(0.451) | 0.287 | <0.001 | 1.74 | 3.26(0.301) | 0.372 | <0.001 | 2.25 | 3.68(0.386) | 0.424 | <0.001 | 2.31 |
| Weight | −0.154(0.063) | −0.096 | 0.016 | 1.71 | −0.122(0.044) | −0.090 | 0.005 | 1.98 | −0.179(0.050) | −0.136 | <0.001 | 1.70 |
| Roll angle | 1.27(0.264) | 0.155 | <0.001 | 1.13 | 1.14(0.134) | 0.212 | <0.001 | 1.19 | 1.04(0.154) | 0.213 | <0.001 | 1.18 |
| Yaw angle | 0.550(0.164) | 0.105 | 0.001 | 1.07 | 0.828(0.097) | 0.199 | <0.001 | 1.04 | 0.620(0.109) | 0.174 | <0.001 | 1.10 |

EP 4 458 266 A1

# FIG. 7

| | Slow (N = 110) | Medium (N = 536) | Fast (N = 113) | All (N = 759) |
|---|---|---|---|---|
| Gait speed | | | | |
| GAITRite | 85.2 ± 9.2 | 114.6 ± 9.4 | 141.6 ± 8.9 | 114.4 ± 17.9 |
| Model 0 | 88.2 ± 10.4 | 114.8 ± 10.1 | 137.8 ± 9.2 | 114.4 ± 16.8 |
| Model 1 | 87.6 ± 10.3 | 114.7 ± 10.0 | 138.9 ± 8.8 | 114.4 ± 17.1 |
| Model 2 | 87.5 ± 10.4 | 114.7 ± 10.1 | 138.9 ± 8.6 | 114.4 ± 17.1 |
| p | <0.001 | 0.728 | <0.001 | 1.00 |
| posthoc | a<b,c,d | | a>c,d>b | |
| ICC | | | | |
| Model 0 | 0.766 ± 0.088* | 0.817 ± 0.027* | 0.665 ± 0.133* | 0.933 ± 0.009* |
| Model 1 | 0.806 ± 0.070* | 0.866 ± 0.020* | 0.753 ± 0.092* | 0.952 ± 0.006* |
| Model 2 | 0.817 ± 0.065* | 0.869 ± 0.019* | 0.778 ± 0.089* | 0.954 ± 0.006* |
| P(b,c) | 0.277 | <0.001 | 0.066 | <0.001 |
| P(b,d) | 0.157 | <0.001 | 0.014 | <0.001 |
| P(c,d) | 0.738 | 0.779 | 0.523 | 0.548 |
| posthoc | | b<c,d | | b<c,d |
| ME | | | | |
| Model 0 | 3.63 | 0.27 | −2.64 | 0.32 |
| Model 1 | 2.92 | 0.13 | −1.85 | 0.24 |
| Model 2 | 2.75 | 0.14 | −1.85 | 0.22 |
| p | 0.047 | 0.462 | 0.009 | 0.691 |
| posthoc | b>d | | b<c,d | |
| MAE | | | | |
| Model 0 | 6.05 | 4.08 | 4.20 | 4.38 |
| Model 1 | 5.68 | 3.57 | 3.32 | 3.84 |
| Model 2 | 5.37 | 3.52 | 3.18 | 3.74 |
| p | 0.043 | <0.001 | <0.001 | <0.001 |
| posthoc | | b>c,d | b>c,d | b>c>d |
| RMSE | | | | |
| Model 0 | 6.86 | 5.89 | 7.71 | 6.34 |
| Model 1 | 6.16 | 5.03 | 6.34 | 5.42 |
| Model 2 | 6.01 | 4.99 | 5.96 | 5.30 |

# FIG. 8

# FIG. 9

1100

GAIT SPEED
ESTIMATION DEVICE

1110

COMMUNICATION
UNIT

1120

PROCESSOR

1130

DB

## INTERNATIONAL SEARCH REPORT

| International application No. |
| --- |
| **PCT/KR2022/020054** |

### A.    CLASSIFICATION OF SUBJECT MATTER

**A61B 5/11**(2006.01)i; **A61B 5/00**(2006.01)i; **A61B 5/16**(2006.01)i; **G16H 50/20**(2018.01)i; **G16H 50/70**(2018.01)i

According to International Patent Classification (IPC) or to both national classification and IPC

### B.    FIELDS SEARCHED

Minimum documentation searched (classification system followed by classification symbols)

A61B 5/11(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 뇌졸중(stroke), 보행 속도(walking speed), 가속도계(accelerometer), 체중심(center of gravity)

### C.    DOCUMENTS CONSIDERED TO BE RELEVANT

| Category* | Citation of document, with indication, where appropriate, of the relevant passages | Relevant to claim No. |
| --- | --- | --- |
| Y | 배원식 등. 뇌졸중 환자에서 기능평가와 보행 및 균형과의 관련성. 대한물리의학회지. vol. 6, no. 1, pp. 59-69, 28 February 2011 (BAE, Won-Sik et al. The Relation Between the Fugl-Meyer Motor Assessment and Walking and Balance Ability in Stroke Patient. Journal of the Korean Society of Physical Medicine).<br>        [Retrieved on 03 February 2023]. Retrieved from <https://koreascience.kr/article/JAKO201117463443670.pdf>.<br>        See pages 60-64. | 1-6,8-12 |
| A |  | 7 |
| Y | 이주현 등. 가속도계를 이용한 뇌졸중 환자의 보행분석. 대한재활의학회지. vol. 28, no. 5, pp. 488-493, 31 December 2004 (LEE, Ju Hyun et al. Gait Analysis Using Accelerometer in Stroke Patients. Journal of Korean Academy of Rehabilitation Medicine).<br>        [Retrieved on 03 February 2023]. Retrieved from <https://www.researchgate.net/profile/Youngho-Kim-14/publication/265295809_Gait_Analysis_Using_Accelerometer_in_Stroke_Patients/links/54080d370cf23d9765ae3602/Gait-Analysis-Using-Accelerometer-in-Stroke-Patients.pdf>.<br>        See pages 489-490; and figure 1. | 1-6,8-12 |

☑ Further documents are listed in the continuation of Box C.          ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **20 March 2023** | **20 March 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
|---|
| **PCT/KR2022/020054** |

**C.     DOCUMENTS CONSIDERED TO BE RELEVANT**

| Category* | Citation of document, with indication, where appropriate, of the relevant passages | Relevant to claim No. |
|---|---|---|
| A | 안보라 등. 스마트폰 어플리케이션을 이용한 뇌졸중 환자의 보행 평가 가능성. 대한고유수용성신경근촉진법학회. vol. 16, no. 1, pp. 67-73, 30 April 2018 (AN, Bo-Ra et al. Potential Use of a Smartphone to Evaluate Gait during Walking in Stroke Patients. PNF and Movement). <br>     [Retrieved on 02 February 2023]. Retrieved from <http://koreascience.or.kr/article/ JAKO201819355173986.pdf>. <br>     See pages 68-72. | 1-12 |
| A | 오용섭 등. 가속도계를 이용한 뇌졸중 환자의 보행 측정. 대한고유수용성신경근촉진법학회. vol. 11, no. 2, pp. 31-40, 31 December 2013 (OH, Yong-Seop et al. Walking Measures with a Tri-axial Accelerometer in Stroke Patients. Journal of the Korean Proprioceptive Neuromuscular Facilitation Association). <br>     [Retrieved on 01 February 2023]. Retrieved from <http://koreascience.or.kr/article/ JAKO201317558398385.pdf>. <br>     See pages 32-38. | 1-12 |
| A | 이진복. 체중심 가속도를 이용한 정상보행과 편마비보행 분석. 연세대학교 대학원 의료공학 협동과정, 의공학전공, 석사학위논문. pp. 1-62, 31 December 2002 (YI, Jin-Bock. Gait Analysis of the Normal and the Hemiplegic Walking Using the Acceleration of the CoM. Master's thesis, Graduate Program in Medical Engineering, the Graduate School Yonsei University). <br>     [Retrieved on 01 February 2023]. Retrieved from <https://ir.ymlib.yonsei.ac.kr/bitstream/2 2282913/137883/1/T007485.pdf>. <br>     See pages 1-47. | 1-12 |

Form PCT/ISA/210 (second sheet) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **J. W. HAN ; T. H. KIM ; K. P. KWAK ; K. KIM ; B. J. KIM ; S. G. KIM ; J. L. KIM ; T. H. KIM ; S. W. MOON ; J. Y. PARK.** Overview of the Korean longitudinal study on cognitive aging and dementia. *Psychiatry investigation,* 2018, vol. 15 (8), 767

- **H.-W. JUNG ; H.-J. YOO ; S.-Y. PARK ; S.-W. KIM ; J.-Y. CHOI ; S.-J. YOON ; C.-H. KIM ; K.-I. KIM.** The Korean version of the FRAIL scale: clinical feasibility and validity of assessing the frailty status of Korean elderly. *The Korean journal of internal medicine,* 2016, vol. 31 (3), 594